# EUROPEAN PATENT SPECIFICATION

(11) **EP 4 178 518 B1**
(45) Date of publication and mention of the grant of the patent: **09.07.2025**
(21) Application number: 21842004.0
(22) Date of filing: 13.07.2021
(51) Int. Cl.: A61H 3/00, A61B 5/11, A61B 5/103, A61B 5/107, A61B 5/00, A61H 1/02

(54) **SINGLE-LOWER-LIMB REHABILITATION EXOSKELETON APPARATUS AND CONTROL METHOD**
REHABILITATIONSEXOSKELETTVORRICHTUNG MIT EINEM UNTEREN TEIL UND STEUERUNGSVERFAHREN
APPAREIL D'EXOSQUELETTE DE RÉÉDUCATION DE MEMBRE INFÉRIEUR UNIQUE ET PROCÉDÉ DE COMMANDE

(30) Priority: 13.07.2020 CN 202010672072
(43) Date of publication of application: 17.05.2023
(73) Proprietor: Angelexo Scientific Co., Ltd, Hangzhou, Zhejiang 311200 (CN)
(72) Inventor: YAN, Feng Hui, Hangzhou, Zhejiang 311200 (CN); LI, Lu Ya, Hangzhou, Zhejiang 311200 (CN); ZHANG, Jian, Hangzhou, Zhejiang 311200 (CN)
(74) Representative: Piotrowicz, Pawel Jan Andrzej
(86) International application number: PCT/CN2021/105892
(87) International publication number: WO 2022/012491

(56) References cited:
- CN-A- 103 505 342
- CN-A- 108 939 436
- CN-A- 109 568 089
- CN-A- 110 812 127
- CN-A- 111 249 116
- CN-A- 111 773 037
- CN-B- 108 939 436
- JP-A- 2009 207 840
- US-A1- 2012 116 258
- US-B2- 10 390 973

## Description

### Cross-Reference to Related Application

This application claims the priority of China's prior application, application No.: 202010672072, application date: July 13, 2020.

### Field of Invention

The invention relates to the technical field of rehabilitation exoskeleton, in particular to the single-lower-limb rehabilitation exoskeleton apparatus.

### Background

Rehabilitation exoskeleton is a kind of wearable rehabilitation apparatus. At present, the rehabilitation exoskeleton usually carries out physical rehabilitation training for patient by controlling the movement of patient's dual lower-limbs. For example, the patient initiates the movement of his/her lower-limbs by operating the controller with upper-limbs, or selecting the preprogramed options. The existing rehabilitation exoskeleton, however, does not provide relearning of the walking gait of the patient, and does not allow patient to control the movement proactively either, furthermore it is deficient in information interaction with the patient, and thus has no solution for individualized physical rehabilitation training.

CN 108 939 436 B discloses an initiative lower limb training system for synergy of a healthy side and an injured side and an operation method of the system. Myoelectricity electrodes are distributed on the leg part on the healthy side and the leg part on the injured side, a sensor and a gyroscope are disposed on the leg part on the healthy side, and a computer is connected with the myoelectricity electrodes, the sensor and the gyroscope separately; an outer skeleton part is arranged on the leg part on the injured side and connected with the computer through a motor, and the computer is used for receiving signals input by the myoelectricity electrodes, the sensor and the gyroscope, adopting the driving motor for driving the outer skeleton part to move and adopting a man-machine interaction interface for achieving mirror neuron induction and visual feedback. By means of the system, the movement speed of the healthy side can be adjusted to adjust the movement of the injured side, and therefore adjustment of overall step speed is achieved; a walking process more similar to that of a healthy person is achieved, a patient can carry out real-time adjustment according to his/her conditions, and the subjective initiative of the patient is exerted to a greater extent. By adopting a pressure sensor and a previous time difference calibration mode, the stability and safety of the system are improved.

### Summary of the Invention

According to an aspect of the invention there is provided a single-lower-limb rehabilitation exoskeleton apparatus as specified in claim 1. Optional features are specified in the dependent claims.

The other features and advantages of the invention will be described subsequently in specifications, and, in part, become apparent from the description or understood through the implementation of the invention. The purposes and advantages of the invention can be realized and obtained by the structure specially pointed out in the description, claims and drawings.

In order to make the above-mentioned purposes, features and advantages of the invention more obvious and easy to understand, the following text gives the preferred embodiment, in combination with the attached drawings, the details are as follows.

### Description of the Drawings

In order to more clearly explain the specific embodiments of the invention or the technical solutions in the prior art, the following will be a brief introduction to the specific embodiments or the drawings needed in the description of the existing technology. Obviously, the drawings described below are some embodiments of the invention. For ordinary technicians in the art, without paying creative effort, and other drawings may also be obtained from these drawings.
Fig. 1 is a structural diagram of the single-lower-limb rehabilitation exoskeleton apparatus provided by the embodiment of the invention;
Fig. 2 is a schematic diagram of the patient's lower-limb provided by an embodiment of the invention;
Fig. 3 is a structural diagram of another single-lower-limb rehabilitation exoskeleton apparatus provided by the embodiment of the invention;
Fig. 4 is a layout diagram of a pressure sensor provided by an embodiment of the invention;
Fig. 5 is a frame diagram of a single-lower-limb rehabilitation exoskeleton apparatus provided by the embodiment of the invention;
Fig. 6 is a flow diagram of the control method of the single-lower-limb rehabilitation exoskeleton apparatus provided by the embodiment of the invention;
Fig. 7 is a kinematic solution diagram provided by the embodiment of the present invention;
Fig. 8 is another kinematic solution diagram provided by an embodiment of the present invention.

Labels: 1 - belt; 2 - thigh fixing bandage; 3 - lower leg fixing bandage; 4 - ankle fixing bandage; 5 - pressure sensor array; 5.1 - first intact pressure sensor; 5.2 - second intact pressure sensor; 5.3 - third intact pressure sensor; 6 - sole support; 7 - intact ankle joint sensor; 8 - leg support; 9 - intact knee joint sensor; 10 - thigh support; 11 - intact hip joint sensor; 100 - controller; 200 - intact lower-limb component; 300 - paretic lower-limb component; 13-1 - intact lower-limb; 13-2 - paretic lower-limb.

### Embodiment

In some embodiments, when the intact lower-limb is in standing or supporting state, the current state data of the intact lower-limb is collected by the intact lower-limb component and sent to the controller. The controller controls the state of the paralytic lower-limb by receiving the state data of the intact lower-limb component and sending the state data, such as standing or supporting, to the paralytic lower-limb component.

In some embodiments, the state may also include gait data in the lifting and the walking states. In brief, the state data comprises the movement trajectory or the rotation value of each joint of the intact lower-limb while walking, e.g., the state data in the lifting state may include the lifting height, and the movement data may comprise changes in rotation value of each joint and the swing amplitude of each joint, and so on. The data is collected through the intact lower-limb component, and sent to the controller. Through calculation or determination, the controller sends the gait state or data to the paralytic lower-limb component to control the gait state or data of the paralytic lower-limb component, and consequently control the gait of the paralytic lower-limb.

In some embodiments, the controller obtains the data of the intact lower-limb and, through calculation, sends the data to the paralytic lower-limb component to control the movement of the paralytic lower-limb. It can be understood that some data of intact lower-limb cannot be directly sent to the paralytic lower-limb without calculation for controlling the movement of the paralytic lower-limb by the paralytic lower-limb component.

In some embodiments, the movement data of the intact lower-limb comprises gait data.

In some embodiments, the controller is used to determine the gait data corresponding to the paralytic lower-limb component according to the movement data of the intact lower-limb, and send the gait data to the paralytic lower-limb component, so as to control the gait of the paralytic lower-limb.

In some embodiments, the gait data comprises one or more of walking step length, walking step height, walking step frequency, paralytic ankle angle value, paralytic knee angle value and paralytic hip angle value.

In some embodiments, the paralytic lower-limb component is used to drive the paralytic lower-limb to move according to the gait data while the intact lower-limb is in the supporting state. The gait data herein is calculated by the controller according to the data of the intact lower-limb.

In some embodiments, the intact lower-limb component comprises an intact ankle joint sensor, an intact knee joint sensor and an intact hip joint sensor; The intact ankle joint sensor is used for collecting the angle value of the intact ankle joint of the patient; The intact knee joint sensor is used for collecting the angle value of the intact knee joint of the patient; The intact hip joint sensor is used for collecting the intact hip joint angle value of the patient.

In some embodiments, the movement data also comprises an intact plantar pressure value; The intact lower-limb component also comprises a plurality of intact pressure sensors, each of which is used to collect the intact plantar pressure value. In some embodiments, the controller is also used to determine the current state of the intact lower-limb according to the intact planter pressure value.

In some embodiments, the state data comprises the planter pressure value of the paralytic lower-limb.

In some embodiments, the paralytic lower-limb component also comprises one or more pressure sensors, which are used to collect the paralytic planter pressure value of the patient.

In some embodiments, the controller is also used to determine the current state of the paralytic lower-limb according to the paralytic plantar pressure value. In this way, the state of the paralytic lower-limb can be better obtained, and the paralytic lower-limb can be better controlled by the controller.

In one embodiment, the paralytic lower-limb component comprises one or more joint drive motors for the paralytic ankle joint, the paralytic knee joint and the paralytic hip joint; Each of the joint drive motor is used for controlling the corresponding joint movement of the paralytic lower-limb to the desired joint angle value according to the gait data determined by the controller.

In one embodiment, the paralytic lower-limb component also comprises a corresponding joint power supply and a corresponding joint power button connected with the joint drive motor; The joint power supply is used for supplying power to the corresponding joint drive motor on the paralytic side; The joint power button is used for changing the ON and OFF states of the corresponding joint power supply based on the operational need.

In one embodiment, the intact lower-limb component and the paralytic lower-limb component comprise their own fixing units; The fixing unit with the intact lower-limb component is used for attaching the intact lower-limb component to the intact lower-limb; The fixing unit in the paralytic lower-limb component is used for attaching the paralytic lower-limb component to the paralytic lower-limb.

In one embodiment, the apparatus also comprises a storage unit.

In one embodiment, the apparatus also comprises a display component composed by a liquid crystal touch screen, a power indicator, and an operation indicator; The liquid crystal touch screen is used for displaying the movement data and the gait data; The power indicator is used for indicating the power consumption of the joint power supply; The operation indicator is used for indicating the operational status of the single-lower-limb rehabilitation exoskeleton apparatus.

In the second aspect, the embodiment of the invention also provides a control method of the single-lower-limb rehabilitation exoskeleton apparatus comprising: determining the state data of the intact lower-limb by using intact lower-limb component and the state data of the paralytic lower-limb by using paralytic lower-limb component; The paralytic lower-limb component is controlled by using the current state data of the intact lower-limb component, so as to control the state of the paralytic lower-limb of the patient.

In some embodiments, the state comprises of the current state data or movement data.

In some embodiments, the current state comprises a lifting state or a supporting state.

In some embodiments, the intact lower-limb component is used to determine the current state of the intact lower-limb and the current state of the paralytic lower-limb is determined by using paralytic lower-limb component.

In some embodiments, the movement data of the intact lower-limb component is obtained while the intact lower-limb is in the lifting state; The gait data is sent to the paralytic lower-limb component to drive the paralytic lower-limb to move according to the gait data when the intact lower-limb component is in a supporting state.

In some embodiments, the movement data comprises one or more of the intact ankle angle value, the intact knee angle value, and the intact hip angle value; The gait data corresponding to the paralytic lower-limb component is determined according to the movement data of the intact lower-limb; Wherein the gait data comprises one or more of the walking step length, walking step height, walking step frequency, ankle angle value, knee angle value and hip angle value on the paralytic side.

In one embodiment, gait data corresponding to the paralytic lower-limb component is determined according to the movement data of the intact lower-limb, such as gait data including the walking step length and walking step height of the patient is determined according to the angle value of the hip joint, the angle value of the knee joint and the angle value of the ankle joint on the intact side; According to the length of the time while the intact lower-limb is in the lifting state, the walking step frequency of the patient is determined; According to the gait data of the patient determined by the movement data of the intact lower-limb, including the walking step length, walking step height and walking step frequency, the gait data corresponding to the paralytic lower-limb component is determined, including the walking step length, the walking step height and the walking step frequency; According to the gait data of the patient determined by the movement data of the intact lower-limb, including the walking step length, walking step height and walking step frequency, the gait data corresponding to the paralytic lower-limb component is determined, including the angle value of the ankle joint, the angle value of the knee joint and the angle value of the hip joint on the paralytic side; Or, the paralytic ankle angle value is determined according to the angle value of the intact ankle joint, the paralytic knee angle value is determined according to the angle value of the intact knee joint, the paralytic hip angle value is determined according to the angle value of the intact hip joint.

This invention, as an embodiment, provides a single-lower-limb rehabilitation exoskeleton apparatus and control methods comprising a controller, an intact lower-limb component and a paralytic lower-limb component connecting communicatively with the controller; The intact lower-limb component is used to be attached to the intact lower-limb, and the paralytic lower-limb component is used to be attached to the paralytic lower-limb of the patient; The controller is used to determine the current state of the intact lower-limb through the intact lower-limb component and the current state of the paralytic lower-limb through the paralytic lower-limb component; The current state comprises the lifting state or the supporting state; The intact lower-limb component is used to collect the movement data of the intact lower-limb while the intact lower-limb is in the lifting state, and send the movement data to the controller; The state data includes one or more of the ankle angle value, knee angle value and hip angle value; The controller is used to determine the gait data of the paralytic lower-limb component according to the movement data of the intact lower-limb, and send the gait data to the paralytic lower-limb component; Gait data comprises one or more of the walking step length, walking step height, walking step frequency, ankle angle value, knee angle value and hip angle value on the paralytic side; The paralytic lower-limb component of is used to drive the paralytic lower-limb to move according to gait data while the intact lower-limb is in the supporting state. While the intact lower-limb is in the lifting state, the above apparatus collects the movement data of the intact lower-limb through the intact lower-limb component, and determines the gait data of the paralytic lower-limb by the controller according to the movement data, so that the paralytic lower-limb of the patient can be controlled by the paralytic lower-limb component according to the gait data, and the patient's proactive gait control is better realized, the information interaction between patient and the single lower-limb rehabilitation exoskeleton apparatus can be realized, and individualized physical rehabilitation training can be realized according to the patient's own movement data.

The other features and advantages of the invention will be described subsequently in specifications, and, in part, become apparent from the description or understood through the implementation of the invention. The purposes and advantages of the invention can be realized and obtained by the structure specially pointed out in the description, claims and drawings.

In order to make the above-mentioned purposes, features and advantages of the invention more obvious and easy to understand, the following text gives the preferred embodiment, in combination with the attached drawings, the details are as follows.

### Description of the Drawings

In order to more clearly explain the specific embodiments of the invention or the technical solutions in the prior art, the following will be a brief introduction to the specific embodiments or the drawings needed in the description of the existing technology. Obviously, the drawings described below are some embodiments of the invention. For ordinary technicians in the art, without paying creative effort, and other drawings may also be obtained from these drawings.

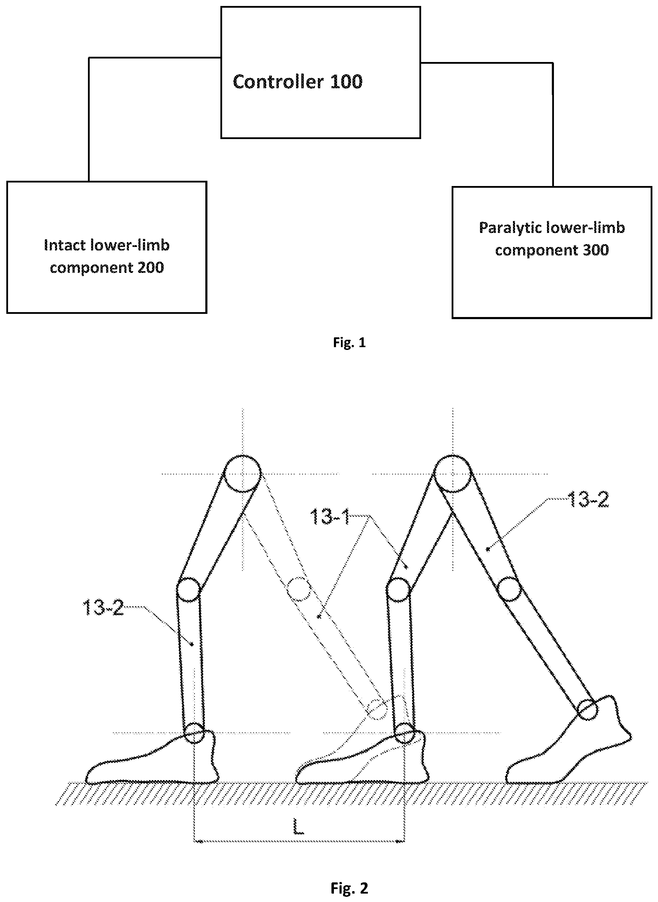

Fig. 1 is a structural diagram of the single-lower-limb rehabilitation exoskeleton apparatus provided by the embodiment of the invention;
Fig. 2 is a schematic diagram of the patient's lower-limb provided by an embodiment of the invention;

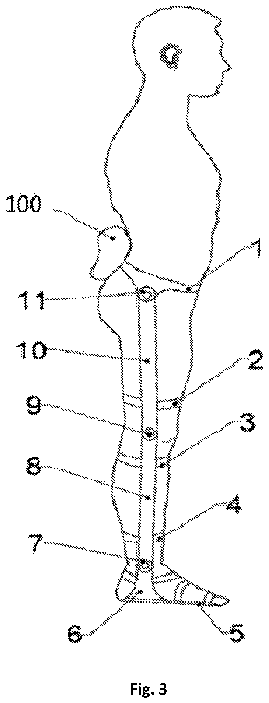

Fig. 3 is a structural diagram of another single-lower-limb rehabilitation exoskeleton apparatus provided by the embodiment of the invention;

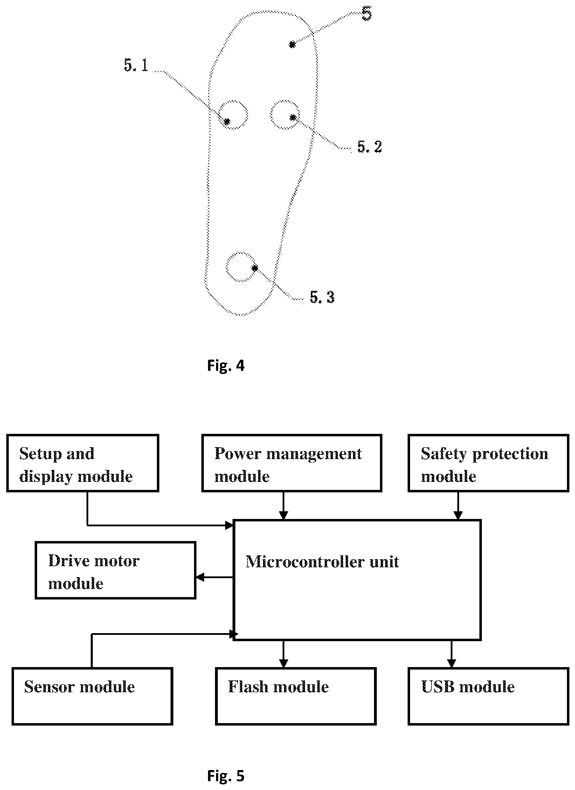

Fig. 4 is a layout diagram of a pressure sensor provided by an embodiment of the invention;
Fig. 5 is a frame diagram of a single-lower-limb rehabilitation exoskeleton apparatus provided by the embodiment of the invention;

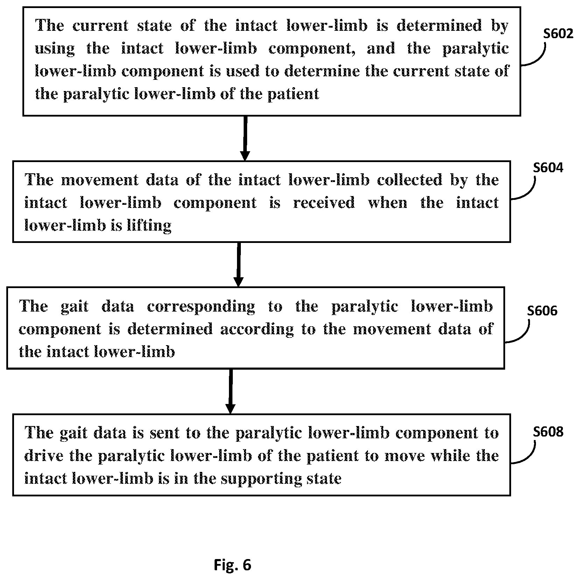

Fig. 6 is a flow diagram of the control method of the single-lower-limb rehabilitation exoskeleton apparatus provided by the embodiment of the invention;

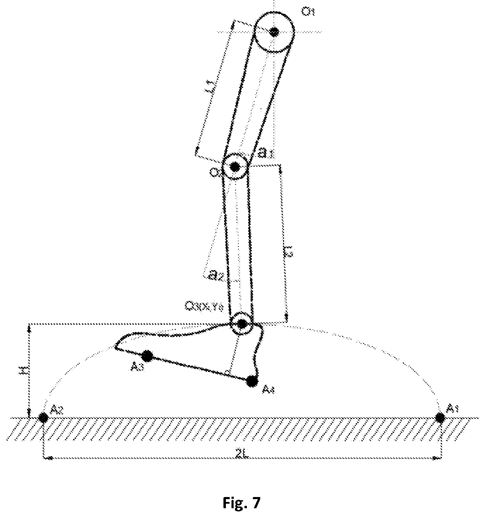

Fig. 7 is a kinematic solution diagram provided by the embodiment of the present invention;

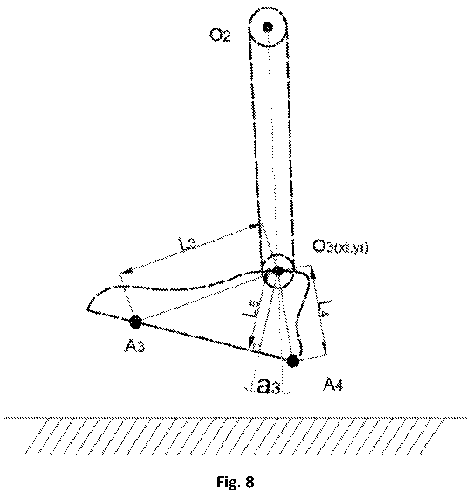

Fig. 8 is another kinematic solution diagram provided by an embodiment of the present invention.

Labels: 1 - belt; 2 - thigh fixing bandage; 3 - lower leg fixing bandage; 4 - ankle fixing bandage; 5 - pressure sensor array; 5.1 - first intact pressure sensor; 5.2 - second intact pressure sensor; 5.3 - third intact pressure sensor; 6 - sole support; 7 - intact ankle joint sensor; 8 - leg support; 9 - intact knee joint sensor; 10 - thigh support; 11 - intact hip joint sensor; 100 - controller; 200 - intact lower-limb component; 300 - paralytic lower-limb component; 13-1 - intact lower-limb; 13-2 - paralytic lower-limb.

### Embodiment

In order to make the purposes, technical solutions and advantages of the embodiments of the invention clearer, the technical solutions of the invention will be described clearly and completely in combination with the embodiments. Obviously, the described embodiments are part of the embodiments of the invention, not all of them. Based on the embodiments in the invention, all other embodiments obtained by ordinary technicians in the art without making creative effort belong to the protection scope of the invention.

At present, the existing rehabilitation exoskeleton robotic products do not provide real-time gait relearning function, i.e., the patient's lower-limbs are completely controlled by the exoskeleton, and the movement of the lower-limbs is controlled by the preprogrammed procedure of the exoskeleton. This training method may not provide the patients with sense of walking and relearning; In addition, the uncomforting mechanical gait control is difficult for patients to accept. In the practice of rehabilitation training, this training method may make the patients experience control difficulties, step disorder, and even the risk of falling; Moreover, because the movement of the intact lower-limb is also controlled by robotic program, the physical rehabilitation of hemiplegia patients is compromised physiologically and psychologically. Based on the above, the implementation of the invention provides a single-lower-limb rehabilitation exoskeleton apparatus and its control methods, which can better realize the patient's proactive gait control and the information interaction between the patient and the single-lower-limb rehabilitation exoskeleton apparatus, thus provide solutions for individualized physical rehabilitation training.

To facilitate the understanding of the embodiment, a single-lower-limb rehabilitation exoskeleton apparatus disclosed in the embodiment of the invention is described first in detail. Referring to the structural diagram of the single-lower-limb rehabilitation exoskeleton apparatus shown in Fig. 1, the apparatus comprises a controller 100, an intact lower-limb component 200 and a paralytic lower-limb component 300 connecting communicatively with the controller 100, wherein the intact lower-limb component 200 is used to be attached to the intact lower-limb of the patient, and the paralytic lower-limb component 300 is used to be attached to the paralytic lower-limb of the patient.

In some embodiments, the controller is used to establish or collect the current state data of the intact lower-limb through the intact lower-limb component, and to determine the current state data of the paralytic lower-limb through the paralytic lower-limb component, for example, the current state may include a lifting state or a supporting state. In one embodiment, the intact plantar pressure value can be collected through the intact lower-limb component to determine the current state of the intact lower-limb according to the intact plantar pressure value. Similarly, the paralytic plantar pressure value can be collected through the paralytic lower-limb component to determine the current state of the paralytic lower-limb according to the paralytic plantar pressure value. In this way, the current state of both the intact and paralytic lower-limbs can be determined and get ready for the next step, also the initial states of both the intact and paralytic lower-limb components can be calibrated to allow for best fitting the patient. The lifting state or supporting state can be detected by the planter pressure sensors.

In some embodiments, the intact lower-limb component is used to collect the state data of the intact lower-limb while the intact lower-limb is in the lifting state, such as movement data, and send the movement data to the controller. The movement data may include one or more of the intact ankle angle value, the intact knee angle value, the intact hip angle value, and the intact plantar pressure value. In one embodiment, the intact lower-limb component can include a plurality of angle sensors, and each angle sensor is respectively set on the intact ankle joint, the intact knee joint and the intact hip joint of the patient, so as to collect the angle value of the intact ankle joint, the angle value of the intact knee joint and the angle value of the intact hip joint of the patient.

The controller is used to determine the corresponding state data of the paralytic lower-limb component, such as gait data, according to the movement data of the intact lower-limb, and send the gait data to the paralytic lower-limb component. Wherein the gait data comprises one or more of walking step length, walking step height, walking step frequency, paralytic ankle angle value, paralytic knee angle value and paralytic hip angle value. In one embodiment, the walking step length and walking step height of the patient can be determined according to the angle value of the intact hip joint, the angle value of the intact knee joint and the angle value of the intact ankle joint. In one embodiment, the walking step frequency of the patient is determined according to the length of time while the intact lower-limb is in the lifting state. According to the movement data (including walking step length, walking step height and walking step frequency) determined by the intact lower-limb movement, the gait data (including walking step length, walking step height and walking step frequency) corresponding to the paralytic lower-limb component is determined. In addition, in some embodiments, the gait data corresponding to the paralytic lower-limb component can be determined according to the gait data (including walking step length, walking step height and walking step frequency) of the patient determined by the intact lower-limb movement, such as the paralytic ankle angle value, the paralytic knee angle value and the paralytic hip angle value; The angle value of the paralytic ankle joint can be determined according to the angle value of the intact ankle joint, the angle value of the paralytic knee joint can be determined according to the angle value of the intact knee joint, and the angle value of the paralytic hip joint can be determined according to the angle value of the intact hip joint. In the specific implementation, one of the above two solutions can be selected based on the actual situation to calculate the angle value of the paralytic ankle joint, the angle value of the paralytic knee joint and the angle value of the paralytic hip joint.

The paralytic lower-limb component is used to drive the paralytic lower-limb to move according to the gait data while the intact lower-limb is in the supporting state. In one embodiment, the paralytic lower-limb component may include a plurality of joint drive motors, and each joint drive motor is arranged respectively on the paralytic ankle joint, knee joint and hip joint of the patient to drive the paralytic ankle joint, knee joint and hip joint of the patient to move according to the gait data. According to the state data of the intact lower-limb, the controller controls these joint drive motors on the paralytic lower-limb component comprising ankle joint, knee joint and hip joint to drive the movement of the paralytic lower-limb.

The single-lower-limb rehabilitation exoskeleton apparatus provided by the embodiment of the invention collects the movement data of the intact lower-limb through the intact lower-limb component while the intact lower-limb is in the lifting state, and determines the gait data of the paralytic lower-limb through the controller according to the movement data, so that the paralytic lower-limb component can control the movement of the paralytic lower-limb according to the gait data, and better realize the patient's proactive gait control and the information interaction between the patient and the single-lower-limb rehabilitation exoskeleton apparatus, so as to provide the solutions for the individualized physical rehabilitation training according to the patient's movement data.

Based on the single-lower-limb rehabilitation exoskeleton apparatus provided by the above embodiment, the intact lower-limb component of the single-lower-limb rehabilitation exoskeleton apparatus provided by the embodiment of the invention also comprises the intact ankle joint sensor, the intact knee joint sensor, the intact hip joint sensor and a plurality of pressure sensors, and the paralytic lower-limb component also comprises the paralytic ankle joint drive motor, the paralytic knee joint drive motor, the paralytic hip joint drive motor and a plurality of pressure sensors. In addition, the intact lower-limb component and the paralytic lower-limb component also include the fixing units, which can include a plurality of bandages.

Wherein the intact ankle joint sensor is used to collect the angle value of the intact ankle joint, the intact knee joint sensor is used to collect the angle value of the intact knee joint, the intact hip joint sensor is used to collect the angle value of the intact hip joint, and each intact pressure sensor is used to collect the intact plantar pressure value.

The paralytic joint drive motors are used to control the corresponding joint movement of the paralytic lower-limb to the desired paralytic joint angle values according to the gait data. Specifically, the paralytic ankle joint drive motor is used to control the ankle joint movement of the paralytic lower-limb to the desired angle value of the paralytic ankle joint according to the gait data, the paralytic knee joint drive motor is used to control the knee joint movement of the paralytic lower-limb to the desired angle value of the paralytic knee joint according to the gait data, and the paralytic hip joint drive motor is used to control the movement of the hip joint of the paralytic lower-limb to the desired angle value of the paralytic hip joint according to the gait data, and each paralytic pressure sensor is used to collect the paralytic plantar pressure value of the patient.

In addition, the controller is also used to determine the current state of the intact lower-limb according to the intact plantar pressure value of the patient and the current state of the paralytic lower-limb according to the paralytic plantar pressure value.

To facilitate the understanding of the above embodiments, the implementation of the invention provides a schematic diagram of the lower-limbs of the patient as shown in Fig. 2, and exemplarily marks the intact lower-limb 13-1 on the intact side and paralytic lower-limb 13-2 on the paralytic side of the patient. The embodiment of the invention takes intact lower-limb 13-1 as an example to further explain the single-lower-limb rehabilitation exoskeleton apparatus. Referring to the structural diagram of the single-lower-limb rehabilitation exoskeleton apparatus shown in Figure 3, the belt 1 is used to attach the controller 100 and one end of the hip joint angle sensor (i.e., the intact hip joint sensor 11) at the appropriate position of the patient's waist, and the other end of the hip joint angle sensor is placed on the thigh supporting mechanism close to the waist, and the hip joint angle sensor is located near the rotation center of the hip joint; One end of the knee joint angle sensor (i.e., the intact side knee joint sensor 9) is placed on the other end of the thigh supporting mechanism close to the sole of the foot, the other end is fixed on the lower leg supporting mechanism close to the waist, and the knee joint angle sensor is located near the knee joint rotation center; One end of the ankle joint angle sensor (i.e., the intact ankle joint sensor 7) is fixed on the other end of the lower leg supporting mechanism close to the sole of the foot, the other end is fixed on the sole supporting mechanism, and the ankle joint angle sensor is located near the rotation center of the ankle joint. In addition, Fig. 3 also shows that the fixing unit with the intact lower-limb component also comprises a thigh fixing bandage 2, a lower leg fixing bandage 3 and an ankle fixing bandage 4, which are used to attach the intact lower-limb component to the intact lower-limb of the patient. Furthermore, it is shown in Fig. 3 that the intact lower-limb component also comprises sole supporting mechanism 6, lower leg supporting mechanism 8, thigh supporting mechanism 10 and other supporting parts.

It should be noted that the fixing unit in the paralytic lower-limb component also comprises thigh fixing bandage, lower leg fixing bandage and ankle fixing bandage, which are used to attach the paralytic lower-limb component to the paralytic lower-limb of the patient. In addition, the paralytic lower-limb component also includes sole supporting mechanism, lower leg supporting mechanism and thigh supporting mechanism.

It can be understood that the controller does not necessarily need to be carried by the patient. When wireless transmission is used, the controller can be operated remotely instead. The data transmission is carried out in the form of wireless communications, such as 5G network.

In addition, the embodiment of the invention provides a schematic diagram of the arrangement of pressure sensors. Exemplarily the intact lower-limb component comprises three intact pressure sensors, as shown in Fig. 4, it constitutes a pressure sensor array 5 which is provided with a first intact pressure sensor 5.1, the second intact pressure sensor 5.2 and the third intact pressure sensor 5.3. The first intact pressure sensor 5.1, the second intact pressure sensor 5.2 and the third intact pressure sensor 5.3 are located at the left front, right front and heel of the sole, respectively.

In practical application, the first intact pressure sensor 5.1, the second intact pressure sensor 5.2, the third intact pressure sensor 5.3, the ankle angle sensor, the knee angle sensor and the hip angle sensor transmit the movement data to the controller through the data line or wireless methods.

Based on the single-lower-limb rehabilitation exoskeleton apparatus provided by the above embodiment, the embodiment of the invention further provides a frame schematic diagram of the single-lower-limb rehabilitation exoskeleton apparatus, as shown in Fig. 5. The single-lower-limb rehabilitation exoskeleton apparatus comprises a microcontroller unit (i.e., the controller), a sensor module, a drive motor module, a setup and display module, a power management module, a control module, a safety protection module, flash module and USB communication module.

In one implementation, microcontroller unit (MCU) can adopt STM32f429 chip based on ARM Cortex-M4 core. Because the STM32f429 chip has Floating Point Unit (FPU) and Digital Signal Processing (DSP) library, it can better perform algorithm operation to meet the requirements for low power consumption and high computing power.

In one embodiment, the setup and display module (i.e., the display component) comprises a liquid crystal touch screen, a power indicator and a status indicator. The liquid crystal touch screen is used to set up and display the movement data and gait data, and the power indicator is used to indicate the power consumption of the joint power supply, for example, the power of the ankle power supply, the knee power supply and the hip power supply respectively, the operation indicator is used to indicate the operation status of the single-lower-limb rehabilitation exoskeleton apparatus. Wherein the power indicator and operation status indicator are red-green dual color light emitting diodes (LED). If the red-green dual color LED is turned on at the same time, it displays in yellow. In the power-on state, the power indicator is red if the power is less than 10%, and green if the power is more than 10%; When the single-lower-limb rehabilitation exoskeleton apparatus is charged, the power indicator is red if the power is less than 10%, yellow if the power is more than 10%, and green if the power is full. The operation status indicator is green in the gait relearning process (i.e., in the normal operation mode) of the single-lower-limb rehabilitation exoskeleton apparatus, and red in case of reaching circuit limit, pressing emergency stop switch, etc. The LCD touch screen is mainly used to set up the thigh length, lower leg length, initial gait characteristic parameters of the paralytic lower-limb. In addition, the LCD touch screen also displays the specific error status, equipment model, software version, etc., to facilitate troubleshooting in case of system failure.

In one embodiment, the drive motor module comprises the drive motors of the paralytic ankle joint, knee joint, and hip joint, which are installed respectively at the ankle joint, knee joint, and hip joint of the paralytic lower-limb component. It can accurately control the joint angle according to the gait relearning algorithm, and has the functions of absolute angle feedback, current feedback, software safety setting, etc.

In one embodiment, the power management module comprises a battery charging management chip, a charging protection circuit, etc., and a customized charger is required for the charging. The paralytic lower-limb component also comprises a corresponding joint power supply and a corresponding joint power button connected with the joint drive motor on the paralytic side, and the joint power supply is used to supply power to the corresponding joint drive motor on the paralytic side; The joint power button is used for changing the ON and OFF states of the corresponding joint power supply based on the operational need. Specifically, taking the paralytic knee joint drive motor and hip joint drive motor as examples, the knee joint power supply connected with the paralytic knee joint drive motor is used to supply power to the paralytic knee joint drive motor, and the hip joint power supply connected with the paralytic hip joint drive motor is used to supply power to the paralytic hip joint drive motor, and the MCU can collect the current power and charging state of knee joint power supply and hip joint power supply. Specifically, the above power supply can adopt lithium batteries.

In one embodiment, the safety protection module is mainly composed of mechanical limit, hardware circuit limit, software limit, emergency stop switch and other safety protection measures, in which the mechanical limit is mainly realized by applying structural limits on the paralytic lower-limb component; The hardware circuit limit can be realized by installing switching circuits. The paralytic lower-limb component also comprises a knee joint power button connected with the paralytic knee joint drive motor, which is used to change the ON an 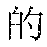 OFF states of the knee joint power supply based on the operational need. The paralytic lower-limb component also comprises a hip joint power button connected with the paralytic hip joint drive motor, which is used to change the ON and OFF states of the power supply of hip joint based on the operational need. In the specific implementation, the hardware circuit limit may cut off the power supply of the drive motor by installing the switching circuit with the joint transmission mechanism. The MCU can only detect the ON and OFF states of the switching circuit, but can not control it; Software limit is to restrict the angle movement after the angle values of hip joint and knee joint on the paralytic side are solved by the gait algorithm; The emergency stop switch may also cut off the power supply of the drive motor directly, and MCU can only detect the ON and OFF state of the emergency stop switch.

In one embodiment, the sensor module may include two absolute encoders and two pressure sensors. For example, two absolute encoders are installed respectively at the hip joint and knee joint of the intact lower-limb to collect the gait trajectory of the intact lower-limb in real time; Two pressure sensors are installed on the soles of the intact lower-limb and the paralytic lower-limb respectively to determine whether the patient is balanced. The feedback data of the pressure sensors will also be integrated into the gait algorithm.

In one embodiment, the flash module (i.e., storage unit) stores basic information of the paralytic lower-limb, such as thigh length, lower leg length and initial gait characteristic parameter, etc., and also records the cumulative gait characteristic parameters of the patient and product logs.

In one implementation, the USB (Universal Serial Bus) module is used to set various parameters, obtain gait characteristics of the patient and query product logs.

The single-lower-limb rehabilitation exoskeleton apparatus provided by the embodiment of the invention is used to control the rehabilitation training for the paralytic lower-limb of the patient by collecting the relevant data from individual patient's own intact lower-limb, so as to enhance the walking comfort and solve coordination problems caused by individual differentiation during the rehabilitation training, In order to achieve the purpose of individualized rehabilitation treatment.

Based on the single-lower-limb rehabilitation exoskeleton apparatus provided in the above embodiment, the embodiment of the invention provides a control method for the single-lower-limb rehabilitation exoskeleton apparatus, which is applied to the single-lower-limb rehabilitation exoskeleton apparatus provided in the above embodiment. Referring to the flow diagram of a control method of the single-lower-limb rehabilitation exoskeleton apparatus shown in Fig. 6, the method mainly comprises the following steps, S602 to S608:
In step S602, the current state of the intact lower-limb is determined by using the intact lower-limb component, and the paralytic lower-limb component is used to determine the current state of the paralytic lower-limb of the patient, wherein the current state comprises the lifting state or the supporting state. In one embodiment, the current state of the intact lower-limb can be determined based on the plantar pressure value which is collected by the pressure sensor with the intact lower-limb component. Specifically, in the walking cycle on the solid and flat ground, while the paralytic lower-limb is standing still, the intact lower-limb steps forward and the intact foot heel is touching the ground slowly; the pressure P₃ᵢ is gradually increased with the third pressure sensor 5.3 located in the intact foot heel. When the intact foot palm is approaching the ground, the first pressure sensor 5.1 and the second pressure sensor 5.2 underneath the intact foot detect the increasing pressures of P₁ᵢ and P₂ᵢ If P₁ᵢ + P₂ᵢ + P₃ᵢ is greater than 1/2 of the body weight and approaching the whole body weight G of the patient, it is indicated that the intact lower-limb is in the supporting state, and the gravity center of the patient is beginning to shift, and the intact lower-limb gradually supports the whole body weight. At this point, the paralytic lower-limb component may start to move. When P₁ᵢ + P₂ᵢ + P₃ᵢ is less than G/2, the intact lower-limb of the patients is in its lifting state.

In step S604, the movement data of the intact lower-limb collected by the intact lower-limb component is received when the intact lower-limb is lifting, wherein the movement data comprises one or more of the angle value of the intact ankle joint, the angle value of the intact knee joint and the angle value of the intact hip joint. In one embodiment, the angle value of the intact ankle joint can be collected by the intact ankle joint sensor, the angle value of the intact knee joint can be collected by the intact knee joint sensor, and the angle value of the intact hip joint can be collected by the intact hip joint sensor.

In step S606, the gait data corresponding to the paralytic lower-limb component is determined according to the movement data of the intact lower-limb, wherein gait data comprises one or more of walking step length, walking step height, walking step frequency, paralytic ankle angle value, paralytic knee angle value and paralytic hip angle value. In one embodiment, the walking step length and walking step height of the patient can be determined according to the angle value of the intact hip joint, the angle value of the intact knee joint and the angle value of the intact ankle joint; The walking step frequency is determined according to the length of time while the intact lower-limb is in lifting state; According to the movement data (including walking step length, walking step height and wallting step frequency) determined by the intact lower-limb movement, the gait data (including walking step, walking height and walking step frequency) corresponding to the paralytic lower-limb component is determined; According to the gait data (including walking step length, walking height and walking step frequency) determined by the intact lower-limb movement data, the gait data corresponding to the paralytic lower-limb component is determined, such as the paralytic ankle angle value, the paralytic knee angle value and the paralytic hip angle value. In another embodiment, the paralytic ankle angle value of the patient can be determined according to the angle value of the intact ankle joint, the paralytic knee angle value of the patient can be determined according to the angle value of the intact knee joint, and the paralytic hip angle value of the patient can be determined according to the angle value of the intact hip joint.

In step S608, the gait data is sent to the paralytic lower-limb component to drive the paralytic lower-limb of the patient to move while the intact lower-limb is in the supporting state. In one embodiment, after receiving the gait data, the paralytic lower-limb component can control the ankle joint of the paralytic lower-limb to move to the desired angle of the paralytic ankle joint according to the gait data through the paralytic ankle joint drive motor, and control the knee joint of the paralytic lower-limb to move to the desired angle of the paralytic knee joint according to the gait data through the paralytic knee joint drive motor, and control the hip joint of the paralytic lower-limb to move to the desired angle of the paralytic hip joint according to the gait data through the paralytic hip joint drive motor.

The control method of the single-lower-limb rehabilitation exoskeleton apparatus provided by the embodiment of the invention collects the movement data of the intact lower-limb through the intact lower-limb component while the intact lower-limb is in the lifting state, and determines the gait data of the paralytic lower-limb through the controller according to the movement data, so that the paralytic lower-limb component can control the movement of the paralytic lower-limb according to the gait data. The information interaction between the patient and the single-lower-limb rehabilitation exoskeleton apparatus is achieved, so as to realize the individualized physical rehabilitation training according to the movement data of the patient.

Based on the above step S606, an embodiment of the present invention provides a specific implementation for determining gait data. First, referring to the kinematic solution diagram shown in Fig. 7, O₁ is the position of the hip joint rotation center; O₂ is the position of the knee joint rotation center ; O₃ is the position of the ankle joint rotation center; A₁ is the place where the foot heel is located on the ground for the previous walking cycle; A₂ is the landing site of the foot heel on the ground during the current walking cycle; A₃ is the contact point between the foot heel and the ground; A₄ is the contact point between the foot palm and the ground; L₁ is the effective length of thigh; L₂ is the effective length of the lower leg; ð₁ is the angle formed between thigh and vertical axis (i.e., the angle value of hip joint on the intact side); ð₂ is the angle formed between the lower leg and thigh (i.e., the knee angle value on the intact side); ð₃ is the angle formed between the normal of the plane of the foot and the axial direction of the lower leg (i.e., the angle value of the ankle joint on the intact side); Xᵢ is the abscissa of O₃ at any time; Yᵢ is the ordinate of O₃ at any time, and the coordinate of O₃ at any time is calculated as follows: Xᵢ = L₁ *sin ð₁ +L₂ * sin(ð₁- ð₂), and Yᵢ = L₁ *cos ð₁ + L₂ * con(ð₁- ð₂)

In practical embodiment, the controller of the single-lower-limb rehabilitation exoskeleton apparatus can store the initial gait data S₀ = f (F₁₀, F₂₀, F₃₀, F₄₀) of the patient. Wherein F₁₀ = f (L₁, ð₁, T_{N1}, t,...) is the function relationship of the thigh length L₁, hip angle ð₁, hip torque T_{N1}, time t and other parameters; F₂₀ = f (L₁, L₂, ð₂, T_{N2}, t,...) is the function relationship of thigh length L₁, lower leg length L₂, knee angle ð₂, knee torque T_{N2}, time t, etc.; F₃₀ = f (L₂, L₃, ð₃, T_{N3}, t,...) is the function relationship of lower leg length L₂, sole length L₃, ankle angle ð₃, ankle torque T_{N3}, time t, etc.; F₄₀ = f (L₃, L₄, L₅, ð₃, P_{N0}, t,...) is the function relationship of the sole length L₃, sole length L₄, sole length L₃, ankle angle ð₃, plantar pressure P_{N0}, time t, etc. Further, the plantar pressure P_{N0} = f(P₁₀, P₂₀, P₃₀), where P₁₀ is the intact plantar pressure value collected by the first pressure sensor 5.1, P₂₀ is the intact plantar pressure value collected by the second pressure sensor 5.2, and P₃₀ is the intact plantar pressure value collected by the third pressure sensor 5.3. In practice, the initial gait parameters S₀ of the above-mentioned single-lower-limb rehabilitation exoskeleton apparatus can be obtained by testing or through clinical trial data.

For any time t = i, the gait data of patient wearing exoskeleton rehabilitation apparatus can be stated as Sᵢ=F(f₁ᵢ, f₂ᵢ, f₃ᵢ, f₄ᵢ), wherein f₁ᵢ = f(L₁, ð₁, T_{N3}, t, ...) is the functional relationship of thigh length L₁, hip joint angle ð₁, hip joint torque T_{N1} and time t ; f₂ᵢ = f(L₁, L₂, ð₂, T_{N2}, t, ...) is the functional relationship of thigh length L1, lower leg length L2, knee joint angle ð₂, knee joint torque T_{N2} and time t; f₃ᵢ = f(L₂, L₃, ð₃, T_{N3}, t, ...) is the functional relationship of lower leg length L2, sole length L₃, ankle joint angle ð₃, ankle joint torque T_{N3} and time t; f₄ᵢ = f(L₃, L₄, L₅, ð₃, P_{N0}, t, ...) is the functional relationship of the patient's sole length L3, sole length L4, sole length L5, ankle angle plantar ð₃, plantar pressure function P_{Ni}, time t and other parameters. In addition, P_{Ni} = f(P₁ᵢ + P₂ᵢ + P₃ᵢ), P₁ᵢ is the intact plantar pressure value collected by the first pressure sensor 5.1 at t = i, P₂ᵢ is the intact plantar pressure value collected by the second pressure sensor 5.2 at t = i, and P₃ᵢ is the intact plantar pressure value collected by the third pressure sensor 5.3 at t = i. When the patient is standing still (i.e., in the supporting state), the intact lower-limb is in the state of standing, and the paralytic lower-limb is in the state of lifting without support, the P₁ᵢ + P₂ᵢ + P₃ᵢ is approximately equal to the patient's weight G.

Furthermore, referring to the schematic diagram of another kinematic solution shown in Fig. 8, and combining Fig. 7 and Fig. 8, the plantar pressure sensor can be used to detect the point of time when the foot is touching the ground, and the time interval between two adjacent touchdowns of the same plantar is the instantaneous gait period T = tⱼ - tᵢ, and further obtain the gait frequencyη=T/60; Taking the maximum absolute value of the abscissa of point O₃ to get the walking step length L, i.e., the walking step length L=|(Xᵢ-Xⱼ)/2|; The absolute value of the maximum value of the ordinate of the O₃ point is the step height H, i.e., the step height H=|Yᵢ-Yⱼ|; The patient's walking speed V=L/T=|(Xᵢ-Xⱼ)/2T|. When the patient is standing still, the vertical line perpendicular to the ground is selected as the reference for joint rotation, the forward rotation of the joint presents positive angle value, and the backward rotation of the joint presents the negative angle value. In a walking cycle, the hip joint angle, knee joint angle, ankle joint angle and other information, as well as the hip joint torque T_{N1}, knee joint torque T_{N2}, ankle joint torque T_{N3}, single step period T, plantar pressure data P_{Ni}, etc., via calculation, the patient's walking step length L, walking step height H, walking speed V, walking step frequency or leg swing frequency η, can be obtained.

Now the patient's target gait data can be obtained as S=Σⁿᵢ₌₀ (S₁+S₂+S₃+...+Sᵢ+...)/n

Wherein S is the average data matrix of the target gait (walking step length L, hip angle knee angle ð₁, knee angleð₂, ankle angle ð₃ and walking swing frequency η, etc.); S₁ is the data matrix of the first gait parameter acquisition (walking step length L, hip angle knee angle ð₁, knee angle ð₂, ankle angle ð₃ and walking swing frequency η, etc.); S2 is the data matrix of the second gait parameter acquisition (walking step length L, hip angle knee angle ð₁, knee angleð₂, ankle angle ð₃ and walking swing frequency η, etc.); Sⱼ is the data matrix of the i-th gait parameter acquisition (walking step length L, hip angle knee angle ð₁, knee angleð₂, ankle angle ð₃ and walking swing frequency η, etc.); n is the number of gait cycles collected.

In practical application, in order to improve the real-time performance of the single-lower-limb rehabilitation exoskeleton apparatus, a light operating system (FreeRTOS) is incorporated with the apparatus, which can make more reasonable and effective use of CPU resources, and better ensure the real-time performance and reliability of the system. In one specific implementation, the single-lower-limb rehabilitation exoskeleton apparatus collects the absolute encoder angles of the intact hip joint and knee joint every 5ms, and after these parameters are processed by average filtering, median filtering and other algorithms, the motion trajectory of the intact ankle joint is solved by the data of thigh length, lower leg length and so on. At the same time, the gait characteristics data of the intact lower-limb (such as walking speed, walking step height, walking step length and ankle joint movement curve characteristics) are analyzed through the kinematic trajectory of intact limb, and these data are fed back to the control algorithm of paralytic lower-limb in real time (the motion trajectory of the paralytic lower-limb is calculated according to the movement characteristics, and the movement angles of hip joint and knee joint of the paralytic lower- limb are solved inversely). In addition, in order to prevent gait disorder or potential falling, pressure sensors are installed with the intact and paralytic lower-limbs of the embodiment to determine whether the patient is stably balanced. The feedback data from the pressure sensors is also incorporated into the gait algorithm.

In addition, in order to improve the safety of the single-lower-limb rehabilitation exoskeleton apparatus, safety measures such as mechanical limit, hardware circuit limit, software limit and emergency stop switch are adopted in the embodiment of the invention to protect the paralytic lower-limb. For details, please refer to the preceding embodiment, and the implementation of the invention will not be repeated here.

It should be noted that in order to ensure the relearning of the patient's personal or individualized gait, a standard set of the gait data is established at the beginning, and then the gait data is updated continuously as the patient walks until it is consistent with the gait characteristics of the paralytic lower-limb of the patient. Therefore, when a different patient is arranged and attached with the same apparatus, an updated needs to be set for the alternative patient by using the LCD screen. When the patient is used initially, the paralytic lower-limb of the single-lower-limb rehabilitation exoskeleton apparatus will walk according to the initial preset gait data (walking step length, hip joint angle, knee joint angle, ankle joint angle, walking step frequency, etc.) at the same time, through the data acquisition system of single-lower-limb rehabilitation exoskeleton apparatus, the relative information of the movement on the intact side of the patient (i.e., the above movement data) are collected and sent to the controller. The controller does analysis on the movement data to obtain the patient's gait data (walking step length, hip joint angle, knee joint angle, ankle joint angle, and walking frequency, etc.), a data acquisition matrix. The controller uses the movement data collected from the intact lower-limb to control the move of the paralytic lower-limb component, so that the walking step length, hip joint angle, knee joint angle, ankle joint angle, and walking step frequency of the paralytic lower-limb are consistent with the intact lower-limb periodically. Also the data acquisition of the movement on the intact side and the data output to the paretic side are in continued processes in real time.

To sum up, the control method of the single-lower-limb rehabilitation exoskeleton apparatus provided by the embodiment of the invention can establish the patient's unique gait control through autonomous relearning, so that the paretic lower-limb and the intact lower-limb can maintain a coordinated gait in line with the patient's own walking characteristics, such as walking speed, walking step height, walking step length and foot movement curve characteristics, which can be more comfortable, fast, reliable and safe to the patient with individualized physical rehabilitation training.

## Claims

1. A single-lower-limb rehabilitation exoskeleton apparatus comprising:
a controller (100);
an intact lower-limb component (200) and a paretic lower-limb component (300) that are connected communicatively with the controller (100);
the intact lower-limb component (200) is to be attached to an intact lower-limb (13-1) of a patient and the paretic lower-limb component is to be attached to a paretic lower-limb (13-2) of the patient respectively;
wherein the controller (100) collects state data of the intact lower-limb (13-1) through the intact lower-limb component (200), and the controller (100) controls the paretic lower-limb component (300) according to the state data of the intact lower-limb (13-1);
wherein the intact lower-limb component (200) comprises one or more of an ankle joint sensor, a knee joint sensor and a hip joint sensor; the ankle joint sensor is used for collecting an angle value of an intact ankle joint of the patient; the knee joint sensor is used for collecting an angle value of an intact knee joint of the patient; the hip joint sensor is used for collecting an angle value of an intact hip joint of the patient;
wherein the paretic lower-limb component (300) comprises one or more joint drive motors for a paretic ankle joint, a paretic knee joint and a paretic hip joint; the joint drive motors are used for controlling movement of corresponding joints of the paretic lower-limb (300) according to joint angle values based on gait data;
wherein both the intact and paretic lower-limb components (200, 300) include a fixing unit;
the fixing unit in the intact lower-limb component is used for attaching the intact lower-limb component to the intact lower-limb of the patient, the fixing unit in the paretic lower-limb component is used for attaching the paretic lower-limb component to the paretic lower-limb of the patient;
wherein the state data comprises movement data, the movement data comprises one or more of the angle value of the intact ankle joint, the angle value of the intact knee joint, and the angle value of the intact hip joint on an intact side and a planter pressure value on a paralytic side;
wherein the gait data comprises one or more of a walking step length, a walking step height, a walking step frequency, a paretic ankle angle value, a paretic knee angle value, a paretic hip angle value and an intact planter pressure value on the intact side;
wherein the controller (100) collects current state data of the intact lower-limb (13-1) and the paretic lower-limb (300) through the intact lower-limb component (200) and the paretic lower-limb component (300) respectively,
wherein the current state data comprises a lifting state or a supporting state;
wherein the intact lower-limb component (200) is used to collect the movement data of the intact lower-limb (13-1) while the intact lower-limb (13-1) is in the lifting state, and send the movement data to the controller (100); the controller (100) determines the gait data corresponding to the paretic lower-limb component (300) according to the movement data of the intact lower-limb (13-1) to control the movement of the paretic lower-limb (13-2),
wherein the paretic lower-limb component (300) is used to drive the paretic lower-limb (13-2) to move according to the gait data while the intact lower-limb (13-1) is in the supporting state;
wherein the intact lower-limb component (200) comprises a first intact pressure sensor (5.1), a second intact pressure sensor (5.2) and a third intact pressure sensor (5.3) located at the left front, right front and heel of a sole of an intact foot, respectively;
wherein while the paralytic lower-limb (13-2) is standing still, the intact lower-limb (13-1) steps forward and the heel of intact foot is touching a ground slowly; a pressure P3i is gradually increased with the third pressure sensor (5.3), when a palm of the intact foot is approaching the ground, the first pressure sensor (5.1) and the second pressure sensor (5.2) underneath the intact foot detect increasing pressures of P1ᵢ and P2i, if P1ᵢ + P2i + P3i is greater than 1/2 of a body weight G and approaching the whole body weight G of the patient, it is indicated that the intact lower-limb (13-1) is in the supporting state, and a gravity center of the patient is beginning to shift, and the intact lower-limb (13-1) gradually supports the whole body weight, at this point, the paralytic lower-limb component (300) starts to move, and when P1ᵢ + P2i + P3i is less than G/2, the intact lower-limb (13-1) of the patient is in its lifting state,
wherein the controller (100) establishes a standard set of the gait data at the beginning, and then the controller (100) updates the gait data continuously as the patient walks until it is consistent with the gait characteristics of the paralytic lower-limb (13-2) of the patient.

2. The apparatus according to claim 1, wherein the gait data comprises the planter pressure value on the paralytic side.

3. The apparatus according to one of claims 1,2, wherein the paretic lower-limb component (300) also comprises one or more pressure sensors on the paretic side, which are used to collect the planter pressure value on the paretic side of the patient; the controller is also used for determining the current state of the paretic lower-limb according to the paretic plantar pressure.

4. The apparatus according to claim 3, wherein the paretic lower-limb component also comprises a corresponding joint power supply and a corresponding joint power button connected with the joint drive motor; the joint power supply is used for supplying power to the corresponding joint drive motor on the paretic side; the joint power button is used for changing ON and OFF states of the corresponding joint power supply based on operational need.

## Patentansprüche

1. Rehabilitationsexoskelettvorrichtung für eine einzelne untere Gliedmaße, umfassend:
eine Steuereinrichtung (100);
eine Komponente (200) für eine intakte untere Gliedmaße und eine Komponente (300) für eine paretische untere Gliedmaße, die kommunikativ mit der Steuereinrichtung (100) verbunden sind;
wobei die Komponente (200) für eine intakte untere Gliedmaße an einer intakten unteren Gliedmaße (13-1) eines Patienten anzubringen ist bzw. die Komponente für eine paretische untere Gliedmaße an einer paretischen unteren Gliedmaße (13-2) des Patienten anzubringen ist;
wobei die Steuereinrichtung (100) Zustandsdaten der intakten unteren Gliedmaße (13-1) durch die Komponente (200) für eine intakte untere Gliedmaße erfasst und die Steuereinrichtung (100) die Komponente (300) für eine paretische untere Gliedmaße gemäß den Zustandsdaten der intakten unteren Gliedmaße (13-1) steuert;
wobei die Komponente (200) für eine intakte untere Gliedmaße einen oder mehrere von einem Sprunggelenksensor, einem Kniegelenksensor und einem Hüftgelenksensor umfasst; der Sprunggelenksensor zum Erfassen eines Winkelwerts eines intakten Sprunggelenks des Patienten verwendet wird; der Kniegelenksensor zum Erfassen eines Winkelwerts eines intakten Kniegelenks des Patienten verwendet wird; der Hüftgelenksensor zum Erfassen eines Winkelwerts eines intakten Hüftgelenks des Patienten verwendet wird;
wobei die Komponente (300) für eine paretische untere Gliedmaße einen oder mehrere Gelenkantriebsmotoren für ein paretisches Sprunggelenk, ein paretisches Kniegelenk und ein paretisches Hüftgelenk umfasst; die Gelenkantriebsmotoren zum Steuern der Bewegung entsprechender Gelenke der paretischen unteren Gliedmaße (300) gemäß Gelenkwinkelwerten basierend auf Gangdaten verwendet werden;
wobei sowohl die Komponente (200, 300) für eine intakte als auch für eine paretische unter Gliedmaße eine Fixiereinheit einschließen; die Fixiereinheit in der Komponente für eine intakte untere Gliedmaße zum Anbringen der Komponente für eine intakte untere Gliedmaße an der intakten unteren Gliedmaße des Patienten verwendet wird, die Fixiereinheit in der Komponente für eine paretische untere Gliedmaße zum Anbringen der Komponente für eine paretische untere Gliedmaße an der paretischen unteren Gliedmaße des Patienten verwendet wird;
wobei die Zustandsdaten Bewegungsdaten umfassen, die Bewegungsdaten eines oder mehrere von dem Winkelwert des intakten Sprunggelenks, dem Winkelwert des intakten Kniegelenks und dem Winkelwert des intakten Hüftgelenks auf einer intakten Seite und einem Plantardruckwert auf einer paralytischen Seite umfassen;
wobei die Gangdaten eines oder mehrere von einer Gehschrittlänge, einer Gehschritthöhe, einer Gehschrittfrequenz, einem Winkelwert eines paretischen Knöchels, einem Winkelwert eines paretischen Knies, einem Winkelwert einer paretischen Hüfte und einem intakten Plantardruckwert auf der intakten Seite umfassen;
wobei die Steuereinrichtung (100) derzeitige Zustandsdaten der intakten unteren Gliedmaße (13-1) und der paretischen unteren Gliedmaße (300) durch die Komponente (200) für eine intakte untere Gliedmaße bzw. die Komponente (300) für eine paretische untere Gliedmaße erfasst;
wobei die derzeitigen Zustandsdaten einen Hebezustand oder einen Stützzustand umfassen;
wobei die Komponente (200) für eine intakte untere Gliedmaße verwendet wird, um die Bewegungsdaten der intakten unteren Gliedmaße (13-1) zu erfassen, während sich die intakte untere Gliedmaße (13-1) in dem Hebezustand befindet, und die Bewegungsdaten an die Steuereinrichtung (100) zu senden; die Steuereinrichtung (100) die Gangdaten, die der Komponente (300) für eine paretische untere Gliedmaße entsprechen, gemäß den Bewegungsdaten der intakten unteren Gliedmaße (13-1) bestimmt, um die Bewegung der paretischen unteren Gliedmaße (13-2) zu steuern;
wobei die Komponente (300) für eine paretische untere Gliedmaße verwendet wird, um die paretische untere Gliedmaße (13-2) so anzutreiben, dass sie sich gemäß den Gangdaten bewegt, während sich die intakte untere Gliedmaße (13-1) in dem Stützzustand befindet;
wobei die Komponente (200) für eine intakte untere Gliedmaße einen ersten intakten Drucksensor (5.1), einen zweiten intakten Drucksensor (5.2) und einen dritten intakten Drucksensor (5.3) umfasst, die an der linken Vorderseite, rechten Vorderseite bzw. Ferse einer Sohle eines intakten Fußes angeordnet sind;
wobei, während die paralytische untere Gliedmaße (13-2) stillsteht, die intakte untere Gliedmaße (13-1) nach vorne tritt und die Ferse des intakten Fußes langsam einen Boden berührt; ein Druck P3i mit dem dritten Drucksensor (5.3) allmählich erhöht wird, wenn sich eine Sohlenfläche des intakten Fußes dem Boden nähert, der erste Drucksensor (5.1) und der zweite Drucksensor (5.2) unter dem intakten Fuß zunehmende Drücke von P1i und P2i detektieren, falls P1i + P2i + P3i größer als 1/2 eines Körpergewichts G ist und sich dem gesamten Körpergewicht G des Patienten nähert, angegeben wird, dass sich die intakte untere Gliedmaße (13-1) in dem Stützzustand befindet und sich ein Schwerpunkt des Patienten zu verschieben beginnt und die intakte untere Gliedmaße (13-1) allmählich das gesamte Körpergewicht stützt, sich an diesem Punkt die Komponente (300) für eine paralytische untere Gliedmaße zu bewegen beginnt, und wenn P1i + P2i + P3i kleiner als G/2 ist, sich die intakte untere Gliedmaße (13-1) des Patienten in ihrem Hebezustand befindet,
wobei die Steuereinrichtung (100) zu Beginn einen Standardsatz der Gangdaten festlegt und dann die Steuereinrichtung (100) die Gangdaten kontinuierlich aktualisiert, während der Patient geht, bis sie mit den Gangcharakteristiken der paralytischen unteren Gliedmaße (13-2) des Patienten konsistent sind.

2. Vorrichtung nach Anspruch 1, wobei die Gangdaten den Plantardruckwert auf der paralytischen Seite umfassen.

3. Vorrichtung nach einem der Ansprüche 1, 2, wobei die Komponente (300) für eine paretische untere Gliedmaße zudem einen oder mehrere Drucksensoren auf der paretischen Seite umfasst, die verwendet werden, um den Plantardruckwert auf der paretischen Seite des Patienten zu erfassen; die Steuereinrichtung zudem zum Bestimmen des derzeitigen Zustands der paretischen unteren Gliedmaße gemäß dem paretischen Plantardruck verwendet wird.

4. Vorrichtung nach Anspruch 3, wobei die Komponente für eine paretische untere Gliedmaße zudem eine entsprechende Gelenkleistungszufuhr und einen entsprechenden Gelenkleistungsknopf umfasst, der mit dem Gelenkantriebsmotor verbunden ist; die Gelenkleistungszufuhr zum Zuführen von Leistung zu dem entsprechenden Gelenkantriebsmotor auf der paretischen Seite verwendet wird; der Gelenkleistungsknopf zum Ändern eines EIN- und AUS-Zustands der entsprechenden Gelenkleistungszufuhr basierend auf einem Betriebsbedarf verwendet wird.

## Revendications

1. Appareil d'exosquelette de rééducation de membre inférieur unique comprenant :
un dispositif de commande (100) ;
un élément de membre inférieur intact (200) et un élément de membre inférieur parétique (300) qui sont connectés en communication avec le dispositif de commande (100) ;
l'élément de membre inférieur intact (200) étant destiné à être fixé à un membre inférieur intact (13-1) d'un patient et l'élément de membre inférieur parétique étant destiné à être fixé à un membre inférieur parétique (13-2) du patient respectivement ;
ledit dispositif de commande (100) collectant des données d'état du membre inférieur intact (13-1) à travers l'élément de membre inférieur intact (200), et ledit dispositif de commande (100) commandant l'élément de membre inférieur parétique (300) suivant les données d'état du membre inférieur intact (13-1) ;
ledit élément de membre inférieur intact (200) comprenant un ou plusieurs capteurs parmi un capteur d'articulation de cheville, un capteur d'articulation de genou et un capteur d'articulation de hanche ; ledit capteur d'articulation de cheville étant utilisé pour collecter une valeur d'angle d'une articulation de cheville intacte du patient ; ledit capteur d'articulation de genou étant utilisé pour collecter une valeur d'angle d'une articulation de genou intacte du patient ; ledit capteur d'articulation de hanche étant utilisé pour collecter une valeur d'angle d'une articulation de hanche intacte du patient ;
ledit élément de membre inférieur parétique (300) comprenant un ou plusieurs moteurs d'entraînement d'articulation pour une articulation de cheville parétique, une articulation de genou parétique et une articulation de hanche parétique ; lesdits moteurs d'entraînement d'articulation étant utilisés pour commander le mouvement des articulations correspondantes du membre inférieur parétique (300) suivant des valeurs d'angle d'articulation basées sur des données de démarche ;
lesdits éléments de membre inférieur intact et parétique (200, 300) comprenant une unité de fixation ; ladite unité de fixation dans l'élément de membre inférieur intact étant utilisée pour fixer l'élément de membre inférieur intact au membre inférieur intact du patient, ladite unité de fixation dans l'élément de membre inférieur parétique étant utilisée pour fixer l'élément de membre inférieur parétique au membre inférieur parétique du patient ;
lesdites données d'état comprenant des données de mouvement, lesdites données de mouvement comprenant une ou plusieurs valeurs parmi la valeur d'angle de l'articulation de cheville intacte, la valeur d'angle de l'articulation de genou intacte et la valeur d'angle de l'articulation de hanche intacte sur un côté intact et une valeur de pression plantaire sur un côté paralytique ;
lesdites données de démarche comprenant une ou plusieurs parmi une longueur de pas de marche, une hauteur de pas de marche, une fréquence de pas de marche, une valeur d'angle de cheville parétique, une valeur d'angle de genou parétique, une valeur d'angle de hanche parétique et une valeur de pression plantaire intacte sur le côté intact ;
ledit dispositif de commande (100) collectant des données d'état courant du membre inférieur intact (13-1) et du membre inférieur parétique (300) à travers l'élément de membre inférieur intact (200) et l'élément de membre inférieur parétique (300), respectivement ;
lesdites données d'état courant comprenant un état de levage ou un état de support ;
ledit élément de membre inférieur intact (200) étant utilisé pour collecter les données de mouvement du membre inférieur intact (13-1) lorsque le membre inférieur intact (13-1) se trouve dans l'état de levage, et envoyer les données de mouvement au dispositif de commande (100) ; ledit dispositif de commande (100) déterminant les données de démarche correspondant à l'élément de membre inférieur parétique (300) suivant les données de mouvement du membre inférieur intact (13-1) pour commander le mouvement du membre inférieur parétique (13-2) ;
ledit élément de membre inférieur parétique (300) étant utilisé pour entraîner le membre inférieur parétique (13-2) à bouger selon les données de démarche alors que le membre inférieur intact (13-1) se trouve dans l'état de support ;
ledit élément de membre inférieur intact (200) comprenant un premier capteur de pression intact (5.1), un deuxième capteur de pression intact (5.2) et un troisième capteur de pression intact (5.3) situés à l'avant gauche, à l'avant droit et au niveau du talon d'une semelle d'un pied intact, respectivement ;
tandis que ledit membre inférieur paralytique (13-2) est immobile, ledit membre inférieur intact (13-1) s'avançant et ledit talon du pied intact touchant lentement le sol ; une pression P₃ᵢ étant progressivement augmentée avec le troisième capteur de pression (5.3), lorsqu'une paume du pied intact s'approche du sol, le premier capteur de pression (5.1) et le deuxième capteur de pression (5.2) sous le pied intact détectant des pressions croissantes de P1i et P2i, si P1i + P2i + P3i est supérieur à 1/2 d'un poids corporel G et s'approche du poids corporel total G du patient, il est indiqué que le membre inférieur intact (13-1) se trouve dans l'état de support, et un centre de gravité du patient commence à se déplacer, et ledit membre inférieur intact (13-1) supportant progressivement le poids corporel total, à ce moment, l'élément de membre inférieur paralytique (300) commençant à bouger, et lorsque P1i + P2i + P3i est inférieur à G/2, le membre inférieur intact (13-1) du patient se trouvant dans son état de levage,
ledit dispositif de commande (100) établissant un ensemble standard de données de démarche au début, et ensuite ledit dispositif de commande (100) mettant à jour les données de démarche de manière continue lorsque le patient marche jusqu'à ce qu'elles soient compatibles avec les caractéristiques de démarche du membre inférieur paralytique (13-2) du patient.

2. Appareil selon la revendication 1, lesdites données de démarche comprenant la valeur de pression plantaire sur le côté paralytique.

3. Appareil selon l'une des revendications 1 et 2, ledit élément de membre inférieur parétique (300) comprenant également un ou plusieurs capteurs de pression sur le côté parétique, qui sont utilisés pour collecter la valeur de pression plantaire sur le côté parétique du patient ; ledit dispositif de commande étant également utilisé pour déterminer l'état courant du membre inférieur parétique selon la pression plantaire parétique.

4. Appareil selon la revendication 3, ledit élément de membre inférieur parétique comprenant également une alimentation électrique d'articulation correspondante et un bouton d'alimentation d'articulation correspondant connecté au moteur d'entraînement d'articulation ; ladite alimentation électrique d'articulation étant utilisée pour fournir de l'électricité au moteur d'entraînement d'articulation correspondant sur le côté parétique ; ledit bouton d'alimentation d'articulation étant utilisé pour changer les états MARCHE et ARRÊT de l' alimentation électrique d'articulation correspondante en fonction des besoins opérationnels.
